(19) Europäisches Patentamt
European Patent Office
Office européen des brevets

(11) **EP 2 228 359 B1**

(12) # EUROPEAN PATENT SPECIFICATION

(45) Date of publication and mention
of the grant of the patent:
**23.10.2013  Bulletin 2013/43**

(51) Int Cl.:
*C07C 41/50* [(2006.01)]     *C07C 43/303* [(2006.01)]
*B01J 29/16* [(2006.01)]     *B01J 29/48* [(2006.01)]

(21) Application number: **10002007.2**

(22) Date of filing: **26.02.2010**

(54) **Process of oxidative conversion of methanol**

Verfahren zur oxidativen Umwandlung von Methanol

Procédé de conversion oxydative de méthanol

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **27.02.2009  CN 200910078391**

(43) Date of publication of application:
**15.09.2010  Bulletin 2010/37**

(73) Proprietors:
• **China Petroleum & Chemical Corporation
  Beijing 100728 (CN)**
• **Research Institute Of Petroleum Processing,
  Sinopec
  Beijing 100083 (CN)**

(72) Inventors:
• **Yu, Peng
  Haidian District
  Beijing 100083 (CN)**
• **Liu, Jingsong
  Haidian District
  Beijing 100083 (CN)**
• **Rong, Junfeng
  Haidian District
  Beijing 100083 (CN)**
• **Shi, Changzhi
  Haidian District
  Beijing 100083 (CN)**
• **Fu, Qiang
  Haidian District
  Beijing 100083 (CN)**
• **Wang, Jin
  Haidian District
  Beijing 100083 (CN)**
• **Zhang, Wei
  Haidian District
  Beijing 100083 (CN)**
• **Zhou, Xuhua
  Haidian District
  Beijing 100083 (CN)**

(74) Representative: **HOFFMANN EITLE
Patent- und Rechtsanwälte
Arabellastrasse 4
81925 München (DE)**

(56) References cited:
**WO-A1-00/29364        DE-A1-102005 027 701
US-A1- 2005 154 226**

**Description**

TECHNICAL FIELD

The present invention relates to a process of conversion of methanol, particularly a method of oxidative conversion of methanol to polyoxymethylene dimethyl ethers.

BACKGROUND OF THE INVENTION

An important aspect in the research of C1 techniques is to produce various useful chemicals from methanol.

For example, dehydration of methanol in the presence of catalyst results in dimethyl ethers, which have good combustion characteristics attributing to its high hexadecane numbers. Dimethyl ethers can be widely used as substitutes for civil liquid petroleum gas and vehicle fuel. In addition, dimethyl ethers can also be used as propellant for aerosol, foaming agent, solvent and extractant etc.

US 6, 166, 266 discloses a method for synthesis of polyoxymethylene dimethyl ethers ($CH_3O (CH_2O)_xCH_3$, $DMM_x$, $2 \leq x \leq 8$) from formaldehyde and dimethyl ether containing material, using borosilicate exhibiting the MFI crystal structure or ion exchange resin having proton acid as catalyst.

EP 1, 505, 049A1 discloses a method for synthesis polyoxymethylene dimethyl ether ($CH_3O (CH_2O)_xCH_3$, $DMM_x$, $2 \leq x \leq 5$) from methylal and polyoxymethylene using fluorosulfonic acid as catalyst.

Polyoxymethylene dimethyl ether ($DMM_x$) with a higher hexadecane number of larger than 60 can be used as engine fuel for compression ignition internal combustion diesel engines or additive for diesel fuel. Further, since polyoxymethylene dimethyl ether ($DMM_x$) is a liquid at ambient temperature, it can be easily stored and transported.

WO 00/29364 relates to the preparation of polyoxymethylene dimethyl ethers by reaction of dimethyl ether with formaldehyde over heterogeneous catalysts. The catalyst comprises silver as an essential component. The catalyst is capable of hydrating dimethyl ether under conditions of reaction sufficient to form an effluent comprising water, methanol, formaldehyde, dimethylether, and polyoxymethylene dimethyl ethers.

US 2005/0154226 describes the oxidation of methanol and/or dimethyl ether using supported molybdenum containing heteropolyacid catalysts. The production of dimethyl ether from methanol can be inhibited by partial deactivation of acid sites on the catalyst.

DE 10 2005027701 relates to a process for preparing polyoxymethylene dimethyl ether from methanol and formaldehyde followed by distillation of the reaction mixture.

SUMMARY OF THE INVENTION

The objective of the invention is to provide a new method for oxidative conversion of methanol.

The present invention provides a new process of preparing polyoxymethylene dimethyl ether from methanol, comprising contact-reacting methanol with oxidant in the presence of a catalyst, wherein the catalyst comprises at least one Group VIB metal component in an amount of from 0.5 to 50 wt% (in terms of metal oxide) and at least one Group VIII metal component in an amount of from 0.2 to 20 wt% (in terms of metal oxide), and at least one molecular sieve having acid catalytic activity in an amount of from 40 to 95 wt%, based on the total weight of the catalyst, and the Group VIII metal component in the catalyst is iron, wherein the oxidant is oxygen or a gas mixture of oxygen with other gases which are inert to methanol.

The present invention further provides a gas-phase process of preparing polyoxymethylene dimethyl ether (DMMx) from methanol, comprising mixing methanol and oxidant to form a mixed gas of methanol and oxidant, feeding the mixed gas into reactor, passing the mixed gas through catalyst bed to form a product mixture containing DMMx, characterized in that, it comprises a step of adjusting the temperature of the reactor under controlled thermal condition

Compared with the prior methods, the process according to the present invention can convert methanol to dimethyl ether and polyoxymethylene dimethyl ether ($DMM_x$, $2 \leq x \leq 8$) through one step.

Brief description of the Drawings:

Figure 1 is a representative schematic view of a process of preparing polyoxymethylene dimethyl ether from methanol in one step according to one embodiment of the present invention.
Figure 2 is a representative schematic view of a process of preparing polyoxymethylene dimethyl ether from methanol in one step according to another embodiment of the present invention.

**DETAILED DESCRIPTION OF THE INVENTION AND PREFERRED EMBODIMENTS**

It must be noted that, as used in the specification and the appended claims, the singular forms "a", "an" and "the" include plural referents unless the context clearly dictates otherwise.

In accordance to the process according to the present invention, the preferred Group VIB metal component in the catalyst is molybdenum, and the preferred Group VIII metal component in the catalyst is iron. The metal components can be salts, oxides, or sulfides thereof, with oxides being preferred. The amount of the Group VIB metal component (in terms of metal oxide) is preferably 2 to 20 wt%, and the amount of the Group VIII metal component (in terms of metal oxide) is preferably 0.2 to 10 wt% based on the total weight of the catalyst.

The molecular sieve having acid catalytic activity is preferably selected from the group consisting of molecular sieve having mesoporous structure, macroporous structure, and a combination thereof. Preferably, the mesoporous molecular sieve is ZSM- 5 molecular sieve, and the macroporous molecular sieve is Y- type molecular sieves. More preferably, the mesoporous molecular sieve is ZSM- 5 having a ratio of $SiO_2/Al_2O_3 \geq 50$, and the macroporous molecular sieve is one or more Y- type molecular sieves of HY, REY, REHY, USY and REUSY. Preferably, the amount of the molecular sieves is 60 to 90 wt%, more preferably 70 to 90 wt%, based on the total weight of the catalyst.

The molecular sieve can be commercially available or can be prepared by any known methods in the art. For example, CN1187462A, CN1121979C and CN1257840C disclose phosphor- or rare earth elements- containing ZSM-5 molecular sieve having different silica alumina ratios and crystal grains and the synthesis methods thereof. CN1005387B, CN1069553C, CN1205915A and CN10610976A disclose HY, REY, REHY, USY and REUSY molecular sieves and the synthesis methods thereof.

There are no particular limitations on processes for preparing the catalyst as long as the conditions are sufficient to load the metal components onto the molecular sieves. For example, one method which can be used to prepare the catalyst comprises: preparing aqueous solution of the compound of the metal component, then impregnating the molecular sieve having acid catalytic activity with the solution, and drying and calcinating/uncalcinating the molecular sieve. The methods and conditions for drying and calcinating are well known for preparing catalysts. Preferably, drying is conducted at a temperature of 50 to 300°C for 0.5 to 12 hours, and more preferably, at a temperature of 100 to 250°C for 1 to 6 hours, and calcination is conducted at a temperature of 350 to 650°C for 0.5 to 12 hours, and more preferably, at a temperature of 400 to 600°C for 1 to 4 hours.

In accordance to the process according to the present invention, the catalysts can be any mould products, such as microspheres, spheres, tablets, or strip etc. Molding can be conducted by conventional methods, such as <u>tablet molding,</u> extruding molding, or rolling sphere molding. Molding can be operated either by molding the molecular sieve followed by loading the molecular sieve with metal components or by mixing the molecular sieve with the metal components followed by molding the mixture, when the catalysts are various kinds of molding products easy to be operated.

Molding can be conducted by conventional methods, such as <u>tablet molding,</u> rolling sphere molding and extruding molding etc. When using the conventional methods, it is permissible to introduce into the mixture adjuvants to ensure smooth molding operation. For example, when <u>molding strips</u> by extrusion, suitable amount of extrusion aids and water can be introduced into the mixture before the extrusion. The categories and amount of the extrusion aids used is conventional in the art. For example, the typical extrusion aids can be selected from the group consisting of sesbania powder, methyl cellulose, starch, polyvinyl alcohol, and a combination thereof.

In accordance to the process according to the present invention, it is preferred to further include heat-resistant inorganic oxide matrix in the catalyst. The amount of the heat-resistant inorganic oxide matrix is no more than 80 wt%, preferably no more than 60 wt% of the total weight of the catalyst.

Said heat-resistant inorganic oxide matrixes are heat-resistant inorganic oxides which are usually used as matrix for catalyst. For example, the heat-resistant inorganic oxides are selected from the group consisting of alumina, silica (silicon oxide), titanium oxide, magnesium oxide, silica-alumina, silica-magnesium oxide, silica-zirconium oxide, silica-thorium oxide, silica-beryllium oxide, silica-titanium oxide, silica-zirconium oxide, titanium oxide-zirconium oxide, silica-alumina-thorium oxide, silica-alumina-titanium oxide, silica-alumina-magnesium oxide, silica-alumina-zirconium oxide, preferably one or more of alumina, silica, silica-alumina, and a combination thereof.

When the catalyst comprises heat-resistant inorganic oxide matrix, there is no particular limitation on the preparation method of the catalyst of the present invention provided that the conditions are sufficient for loading the metal components into the mixture of the molecular sieve and the heat-resistant inorganic oxide matrix.

Preferred preparation method comprises the following steps:

( 1 ) mixing the molecular sieve with the heat-resistant inorganic oxide matrix and/or the precursor thereof;
( 2 ) formulating aqueous solution of compound containing the metal components;
( 3 ) impregnating the mixture of the step (1) with the solution of the step (2), followed by drying, calcining or not calcining the mixture.

The drying and calcinating methods and conditions thereof are commonly known in the art. Preferably, drying is performed at a temperature of 50 to 300°C for a period of 0.5 to 12 hours, and more preferably, at a temperature of 100 to 200°C for a period of 1 to 6 hours. Preferred conditions of calcination are at temperature of 350 to 600°C for 0.5 to 8 hours, and more preferred are at temperature of 400 to 500°C for 1 to 4 hours.

When the catalyst is prepared by mixing the precursor of the heat-resistant oxide matrix and the molecular sieve, it is preferred to include a calcinating step after the mixing step. The methods for calcination and the conditions thereof are those typically used in the preparation of catalysts. Preferably, drying is conducted at a temperature of 50 to 300°C for a period of 0.5 to 12 hours, and more preferably, at a temperature of 10 to 200°C for a period of 1 to 6 hours. Preferred conditions of calcinating is conducted at a temperature of 350 to 600°C for 0.5 to 8 hours, and more preferred at a temperature of 400 to 500°C for 1 to 4 hours.

In accordance to the process according to the present invention, the oxidant is oxygen or the gas mixture of oxygen and other gases which are inert to methanol in the above conditions. The gas which is inert to methanol is preferably selected from the group consisting of air, nitrogen, inert gases, and a combination thereof. The oxygen content of the gas mixture is 1 to 30% by volume.

In accordance to the invention, there is no particular limitation on the reactor provided that the methanol and the oxidant are sufficiently contact-reacted under the above conditions. For example, the reactors can be batch tank reactor, fixed-bed reactor, fluidized-bed reactor, etc. Preferably, the conditions for contact-reaction is as follows: a reaction temperature of 50 to 500°C, preferably 100 to 400°C, more preferably 100 to 300°C, most preferably 200 to 300°C, a reaction pressure of 0.1 MPa to 5 MPa, preferably 0.1 MPa to 5 MPa, more preferably 0.1 MPa to 2 MPa, a mass space velocity of methanol feed of 0.5 to $50h^{-1}$, preferably 3 to $30h^{-1}$, more preferably 8 to 20 $h^{-1}$, and the amount of oxidant are adjusted so that the molar ratio of oxygen to methanol in the contact-reaction is 0.01 to 0.5, preferably 0.05 to 0.3 .

The present invention provides a gas-phase process of preparing polyoxymethylene dimethyl ether (DMMx) from methanol, comprising mixing methanol and oxidant to form a binary mixed gas of methanol and oxidant, feeding the mixed gas into reactor, passing the mixed gas through a catalyst bed to form a product mixture containing DMMx, characterized in that, it comprises a step of adjusting the temperature of the reactor under controlled thermal condition.

The step of adjusting the temperature of the reactor under controlled thermal condition may be varying the mass space velocity of methanol feed. The step of adjusting the temperature of the reactor under controlled thermal condition may be cooling with cooling medium outside the reaction bed, wherein the cooling medium is one or more air, water or heat transfer oil. The step of adjusting the temperature of the reactor under controlled thermal condition may be controlling the cycle amount of non-DMMx portions in the product mixture. Said controlling the cycle amount of non-DMMx portions in the product mixture includes partly or completely incorporating non-DMMx portions in the product mixture into catalyst bed directly from the top of the reactor or at multi-points in separate stage, and controlling the temperature of cycled product under 0-150°C. The ratio of the amount of the cycle product to the starting methanol is from 0.1 to 100. The combination of the above procedures may be used.

The oxidant is oxygen or the gas mixture of oxygen and other gases which are inert to methanol in the above conditions. The gas which is inert to methanol is preferably selected from the group consisting of air, nitrogen, inert gases, and a combination thereof. The oxygen content of the gas mixture is about 1 to about 30% by volume, when the oxidant is a gas mixture of oxygen and other gases which are inert to methanol in the above conditions.

There is no particular limitation on the reactor provided that the methanol and the oxygen containing gases are sufficiently contact-reacted under the above conditions. For example, the reactors can be batch tank reactor, fixed-bed reactor, fluidized-bed reactor, etc. Preferably, the conditions for contact-reaction is as follows: a reaction temperature of 50 to 500°C, preferably 100 to 400°C, more preferably 100 to 300°C, most preferably 200 to 300°C, a reaction pressure of 0.1 MPa to 10 MPa, preferably 0.1 MPa to 5 MPa, more preferably 0.1 MPa to 2 MPa, a mass space velocity of methanol feed of 0.5 to $50h^{-1}$, preferably 3 to $30h^{-1}$, more preferably 8 to 20 $h^{-1}$, and the amount of oxidant are adjusted so that the molar ratio of oxygen to methanol in the contact-reaction is 0.01 to 0.5, preferably 0.05 to 0.3.

The catalyst bed is fixed bed, and the catalyst comprises at least one Group VIB metal component, at least one Group VIII metal component, and at least one molecular sieve having acid catalytic activity.

The product mixture containing DMMx may be feed into separation procedure. According to the process of the invention, the product mixture comprises dimethyl ethers, polyoxymethylene dimethyl ethers and water. It is optional to include one or more operational units of flash evaporation, atmospheric distillation and vacuum distillation to achieve desire separation.

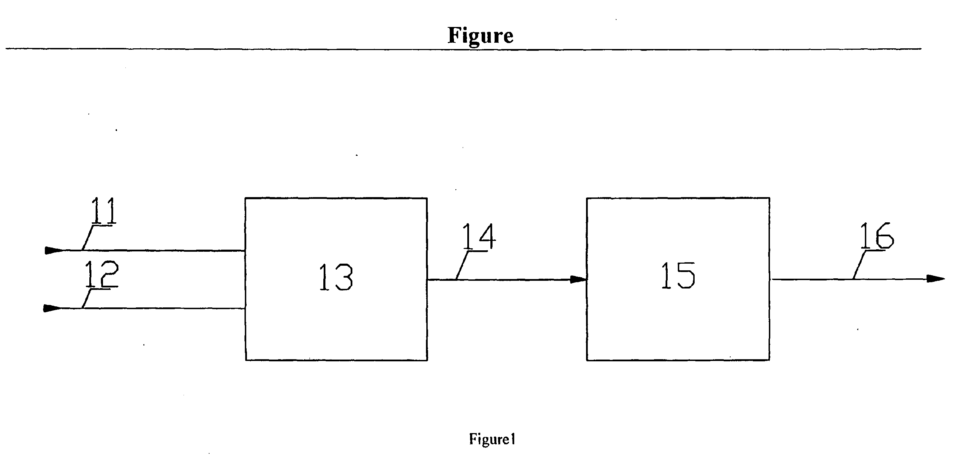

Referring to Figure 1, further description is made about the process according to the present invention. Purified methanol (satisfying GB338-2004 first class) from outside and air are feed into methanol oxidation procedure 13 via pipelines 11 and 12 respectively at the top of the reactor, and are reacted under the temperature of 200°C to 228°C to produce mixed gas containing polyoxymethylene dimethyl ethers. The produced gas is quenched down to 100-120°C in the quenching stage of the reactor to prevent the reaction product from being thermal degraded or prevent intermediate product formaldehyde from being polymerized into polyformaldehyde. The cooled reaction product 14 is feed into product separation procedure 15 and a part of methanol may be feed into product separation procedure as absorbing liquid. In the product separation procedure, polyoxymethylene dimethyl ethers are absorbed by the cycle liquid flowing from the top to the bottom of the column, and the remaining tail gas containing trace amount of polyoxymethylene dimethyl ethers and formaldehyde are passed to the tail gas processor (unshown) and burned. Methanol, dimethyl ether, formaldehyde, methylal, and water in polyoxymethylene dimethyl ethers are vaporized in the product separation step, in which dimethyl ether is used as inert medium and recycled to the conversion part of methanol. It controls the distillation range and flash point of the object product during the product separation step to obtain polyoxymethylene dimethyl ethers product 16 having diesel oil-like properties.

In accordance with the procedure shown by Figure 1, methanol and oxidant are feed into methanol conversion reaction unit 13 via 11 and 12 respectively. The reaction product is feed into separation unit 15 via 14 to obtain dimethyl ether, formaldehyde, methylal, water, and polyoxymethylene dimethyl ethers. The products are removed via 16. The reaction unit 13 comprises quenching exchanger, in which reaction product is quenched to be lower than the temperature under which polyoxymethylene dimethyl ethers is degraded or intermediate product formaldehyde is polymerized into polyformaldehyde, such as from 100-120°C, so as to prevent polyoxymethylene dimethyl ethers from being thermal degraded or prevent intermediate product formaldehyde from being polymerized into polyformaldehyde. Then the reaction product is feed into product separation unit 15 via 14.

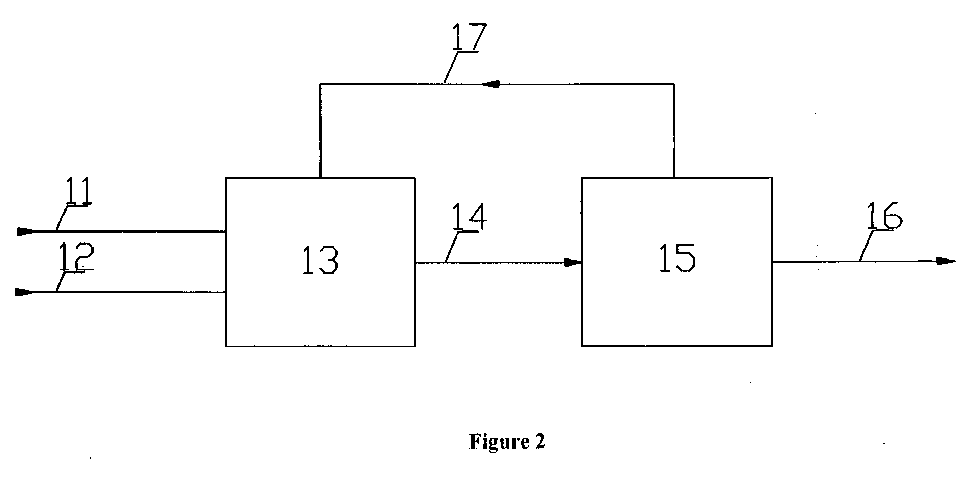

Another embodiment according to the present invention is conducted according to the procedure shown by Figure 2.

In accordance with the procedure shown by Figure 2, a step 17 for cycling at least a portion of non-DMMx in the reaction product to reaction unit 13 is added, with the remaining is the same as that of Figure 1.

The present invention will be further illustrated with reference to the following examples.

Examples 1-7 illustrate the catalysts which can be used in the process of the invention and the method of preparing the same.

Example 1

51g of ammonium molybdate and 5g of ferric nitrate were dissolved in 3L de-ionized water to form a solution, followed by adding to the solution 700 g of ZSM-5 molecular sieve (a $SiO_2/Al_2O_3$ (in mole) of 50, available from Changling Catalyst Plant). After 5 hours, the mixture was filtered and dried at 200°C for 2 hours, and calcined at 500°C for 1 hour, resulting in catalyst C1. The components of the catalyst C1 are reported in Table 1.

Example 2

51g of ammonium molybdate and 5g of ferric nitrate were dissolved in 3L de-ionized water to form a solution, followed by adding to the solution 700 g of USY molecular sieve (available from Changling Catalyst Plant). After 5 hours, the mixture was filtered and dried at 200°C for 2 hours, and calcined at 500°C for 1 hour, resulting in catalyst C2. The components of the catalyst C2 are reported in Table 1.

Example 3

51g of ammonium molybdate and 5g of ferric nitrate were dissolved in 3L de-ionized water to form a solution, followed by adding to the solution 700 g of REY molecular sieve (a rare earth content of 6wt%, available from Changling Catalyst Plant). After 5 hours, the mixture was filtered and dried at 200°C for 2 hours, and calcined at 500°C for 1 hour, resulting in catalyst C3. The components of the catalyst C3 are reported in Table 1.

Example 4

80g of ammonium molybdate and 4g of ferric nitrate were dissolved in 3L de-ionized water to form a solution, followed by adding to the solution 700 g of REY molecular sieve (a rare earth content of 6wt%, available from Changling Catalyst Plant). After 5 hours, the mixture was filtered and dried at 200°C for 2 hours, and calcined at 500°C for 1 hour, resulting in catalyst C4. The components of the catalyst C4 are reported in Table 1.

Example 5

51g of ammonium molybdate and 5g of ferric nitrate were dissolved in 3L de-ionized water to form a solution, followed by adding to the solution 700 g of REY molecular sieve (a rare earth content of 6wt%, available from Changling Catalyst Plant) while stirring at 30°C for 5 hours. Then the catalyst was placed in an oven at 200°C for 2 hours, resulting in catalyst C5. The components of the catalyst C5 are reported in Table 1.

Example 6

51g of ammonium molybdate and 5g of ferric nitrate were dissolved in 3L de-ionized water to form a solution, followed by adding to the solution 700 g of HY molecular sieve (available from Changling Catalyst Plant). After 5 hours, the mixture was filtered and dried at 200°C for 2 hours, and calcinated at 500°C for 1 hour, resulting in catalyst C6. The components of the catalyst C6 are reported in Table 1.

Example 7

700g of REY molecular sieve (a rare earth content of 6 wt%, available from Changling Catalyst Plant) and 10 wt% of $Al_2O_3$ (based on the molecular sieve) were mixed and extrusion molded into strips by a columned orifice-plate having a diameter of 1.2mm. The wet strips were dried at 120°C for 3 hours, and calcinated at 500°C for 2 hours, resulting in a catalyst carrier. When the temperature of the carrier was dropped to room temperature, it was impregnated with 3L aqueous solution containing 51 g of ammonium molybdate and 5g of ferric nitrate for 5 hours. Then, the carrier was filtered and dried at 200°C for 2 hours, and calcinated at 500°C for 1 hour to obtain catalyst C7. The components of the catalyst C7 are reported in Table 1.

Example 8

700g of REY molecular sieve (a rare earth content of 6 wt%) and 30 wt% of $SiO_2$- $Al_2O_3$ (based on the molecular sieve) were mixed to form a mixture of molecular sieve and $SiO_2$- $Al_2O_3$. 51g of ammonium molybdate and 5g of ferric nitrate were dissolved in 3L de- ionized water to form a solution, into which the mixture of molecular sieve and $SiO_2$-$Al_2O_3$ were added. After 5 hours, the mixture was filtered and dried at 200°C for 2 hours, and calcinated at 500°C for 1 hour to obtain catalyst C8. The components of the catalyst C8 are reported in Table 1.

Example 9

102g of ammonium molybdate and 10g of ferric nitrate were dissolved in 3L de-ionized water to form a solution, followed by adding to the solution 700 g of REY molecular sieve (a rare earth content of 6 wt%, available from Changling Catalyst Plant). The mixture was stirred at 30°C for 5 hours. Then the catalyst was placed in an oven at 200°C for 2 hours to obtain catalyst C9. The components of the catalyst C9 are reported in Table 1.

Table 1

| Example | Catalyst | Molecular Sieve, % | Matrix, % | Molybdenum oxide, % | Ion oxide , % |
|---|---|---|---|---|---|
| 1 | C1 | 86.48 | 0 | 8.40 | 5.12 |
| 2 | C2 | 90.18 | 0 | 8.36 | 1.46 |
| 3 | C3 | 90.71 | 0 | 8.67 | 0.62 |
| 4 | C4 | 87.57 | 0 | 11.96 | 0.47 |
| 5 | C5 | 91.83 | 0 | 7.61 | 0.56 |
| 6 | C6 | 95.08 | 0 | 4.57 | 0.35 |
| 7 | C7 | 83.60 | 8.36 | 7.62 | 0.42 |
| 8 | C8 | 71.16 | 21.35 | 7.12 | 0.37 |
| 9 | C9 | 75.42 | 0 | 17.06 | 7.52 |

Example 10

In accordance with the procedure shown by Figure 2, a conversation of methanol is conducted, wherein the feed of methanol is a methanol having first class under GB338-2004, the reactor is a shell and tube fixed reactor, the cooling medium is water and the catalyst is C5.

The feeding amount of methanol is 12000kg/h, the conversion rate of methanol in one-run is more than 96-98%, the output amount of DMMx is 4080kg/h and the cycling dimethyl ether is in an amount of 10000kg/h. The other reaction conditions are listed in Table 2, and the reaction product is listed in Table 3. The result shows the selectivity of DMMx may be up to 34.6% under the above operating conditions.

Table 2 reactor conditions

| reaction conditions | |
| --- | --- |
| Temperature | 250°C |
| Pressure | 1.0 MPa(G) |
| Oxygen/methanol | 0.3 |
| Conversion rate of methanol in one-run | 98 |
| Selectivity of DMMx | 33 |
| Weight space velocity(h$^{-1}$) | 12 |

Table 3 the composition of the product feeding into the absorbing system

| Composition of products | |
| --- | --- |
| DMMx | 34% |
| Methanol | 2% |
| formaldehyde | 3% |
| methylal | 3% |
| Water | 5% |
| Dimethyl ether | 23% |
| Carbon monooxide | 8% |
| Carbon dioxide | 22% |

Examples 11-24 illustrate the process provided in the invention and their technical effect.

The reactions took place in a fixed-bed reactor. The methanol is an analytical reagent available from Beijing Chemical Works, and the oxidant is air. Catalysts C1-C6 and C9 were pressed into tablets, broken and sieved, resulting in particles of 20-40 meshes. Catalysts C7-C8 were broken and sieved, resulting in particles of 20-40 meshes.

Table 4 summarized the catalyst used in each example and reaction conditions.

2 hours after the reaction, samples were taken out for analysis in Agilent 6890 Chromatography.

$$\text{Methanol conversion} = ((MOH_{\text{before reaction}} - MOH_{\text{after reaction}})/MOH_{\text{before reaction}}) \times 100\%$$

$$\text{Selectivity of polyoxymethylene dimethyl ether} = (DMM_X/(MOH_{\text{before reaction}} - MOH_{\text{after reaction}})) \times 100\%$$

The results are reported in Table 5.

Comparative Examples 1~2

The molecular sieves of Example 1 and Example 4 were pressed into tablets, broken, and sieved into particles of 20-40 meshes. Evaluations were made according to the reaction conditions of Example 11. The results are reported in Table 5.

Table 4

| Example | Catalyst | Temperature, °C | Pressure, MPa | Space Velocity, h$^{-1}$ | Oxygen/Methanol |
| --- | --- | --- | --- | --- | --- |
| 11 | C1 | 250 | 0.3 | 13 | 0.30 |
| 12 | C2 | 150 | 0.3 | 10 | 0.15 |

(continued)

| Example | Catalyst | Temperature, °C | Pressure, MPa | Space Velocity, h$^{-1}$ | Oxygen/Methanol |
|---------|----------|------------------|----------------|---------------------------|------------------|
| 13 | C2 | 250 | 0.4 | 13 | 0.20 |
| 14 | C3 | 250 | 1.1 | 13 | 0.20 |
| 15 | C3 | 250 | 0.3 | 13 | 0.25 |
| 16 | C4 | 250 | 0.5 | 13 | 0.20 |
| 17 | C5 | 250 | 0.4 | 13 | 0.20 |
| 18 | C5 | 250 | 1.6 | 13 | 0.30 |
| 19 | C6 | 350 | 0.3 | 20 | 0.10 |
| 20 | C6 | 250 | 0.2 | 13 | 0.20 |
| 21 | C7 | 250 | 1.6 | 13 | 0.10 |
| 22 | C8 | 300 | 1.1 | 13 | 0.10 |
| 23 | C9 | 250 | 1.1 | 13 | 0.20 |
| 24 | C9 | 250 | 0.3 | 13 | 0.20 |

Table 5

| Example | Catalyst | Methanol Conversion/% | DMMx Selectivity/% |
|---------|----------|------------------------|---------------------|
| 11 | C1 | 83.4 | 7.1 |
| 12 | C2 | 81.4 | 15.9 |
| 13 | C2 | 82.9 | 16.2 |
| 14 | C3 | 97.2 | 26.4 |
| 15 | C3 | 96.0 | 26.1 |
| 16 | C4 | 97.6 | 31.7 |
| 17 | C5 | 97.1 | 26.6 |
| 18 | C5 | 98.4 | 34.1 |
| 19 | C6 | 87.1 | 10.9 |
| 20 | C6 | 84.9 | 11.7 |
| 21 | C7 | 94.1 | 21.4 |
| 22 | C8 | 89.2 | 17.2 |
| 23 | C9 | 97.4 | 27.9 |
| 24 | C9 | 93.5 | 24.2 |
| Comparative Example 1 | ZSM-5 | 60.9 | 0 |
| Comparative Example 2 | REY | 94.7 | 5.1 |

Compared with the referenced methods, the process according to the present invention, especially that in Example 17 can convert methanol directly to dimethyl ether and polyoxymethylene dimethyl ether (DMM$_X$ , 2≤x≤8 ) by one step. The methanol conversion reaches 98.4% and DMMx selectivity reaches 34.1% when using REY as molecular sieve in the catalyst. It is particularly suitable for preparing procedure requiring high yield of DMMx.

**Claims**

1. A process for preparing polyoxymethylene dimethyl ether from methanol, comprising contact-reacting methanol with an oxidant in the presence of a catalyst, **characterized in that**, the catalyst comprises at least one Group VIB an metal component in an amount of from 0.5 to 50 wt% (in terms of metal oxide) and at least one Group VIII metal component in an amount of from 0.2 to 20 wt% (in terms of metal oxide), and at least one molecular sieve having acid catalytic activity in an amount of from 40 to 95 wt%, based on the total weight of the catalyst, and the Group VIII metal component in the catalyst is iron, wherein the oxidant is oxygen or a gas mixture of oxygen with other gases which are inert to methanol.

2. The process of claim 1, **characterized in that**, the molecular sieve having acid catalytic activity is selected from the group consisting of molecular sieve having mesoporous structure, macroporous structure, and a combination thereof, wherein the amount of the molecular sieve is 70 to 90 wt% based on the total weight of the catalyst.

3. The process of claim 2, **characterized in that**, the molecular sieve with mesoporous structure is ZSM-5 molecular sieve, and the molecular sieve with macroporous structure is Y-type molecular sieve, preferably the Y-type molecular sieve is selected from the group consisting of HY, REY, REHY, USY, REUSY, and a combination thereof, more preferably the Y-type molecular sieve is selected from the group consisting of REY, REHY, REUSY and a combination thereof.

4. The process of claim 1, **characterized in that**, the catalyst further comprising heat-resistant inorganic oxide matrix in an amount of no more than 80 wt%, preferably no more than 60 wt%, based on the total weight of the catalyst, preferably the heat-resistant inorganic oxide matrix is selected from the group consisting of alumina, silica, silica-alumina, and a combination thereof.

5. The process of claim 1, **characterized in that**, the conditions for contact-reaction comprises a temperature within the range of from 50°C to 500°C, preferably from 100 to 400°C, more preferably from 100 to 300°C, a pressure within the range of from 0.1 MPa to 10 MPa, preferably from 0.1 MPa to 5 MPa, more preferably from 0.1 MPa to 2 MPa, and a mass space velocity of methanol feed within the range of from 0.5 $h^{-1}$ to 50$h^{-1}$, preferably from 3 to 30$h^{-1}$, more preferably from 8 to 20$h^{-1}$, and the content of the oxidant is selected such that the molar ratio of oxygen to methanol in the contact-reaction is 0.01 to 0.5, preferably from 0.05 to 0.3.

6. The process of claim 1, **characterized in that**, the gas which is inert to methanol is one or more gases selected from nitrogen and inert gases.

7. The process of claim 6, **characterized in that**, the oxygen content in the gas mixture is 1 to 30%.

8. The process of claim 1, **characterized in that** it comprises a gas-phase process of preparing polyoxymethylene dimethyl ether (DMMx) from methanol, comprising mixing methanol and the oxidant to form a binary mixed gas of methanol and the oxidant, feeding the mixed gas into a reactor, passing the mixed gas through a catalyst bed to form a product mixture containing DMMx and it comprises a step of adjusting the temperature of the reactor under controlled thermal condition.

9. The process of claim 8, **characterized in that**, the step of adjusting the temperature of the reactor under controlled thermal condition comprises controlling the cycle amount of non-DMMx portions in the product mixture to adjust the reaction temperature.

10. The process of claim 9, **characterized in that**, controlling the cycle amount of non-DMMx parts in the product mixture includes incorporating non-DMMx portions in the product mixture into catalyst bed directly from the top of the reactor or at multi-points in separate stage, and controlling the temperature of cycled product under 0-150°C.

11. The process of claim 9, **characterized in that**, the ratio of the amount of the cycled product to starting methanol is from 0.1 to 100.

12. The process of claim 8, **characterized in that**, the step of adjusting the temperature of the reactor under controlled thermal condition comprises cooling with cooling medium outside the reaction bed, preferably the cooling medium is air, water or heat transfer oil.

**13.** The process of claim 8, **characterized in that**, it further comprising feeding the product mixture containing DMMx into separation procedure.

**14.** The process of claim 8, **characterized in that**, the catalyst bed is fixed bed.

**Patentansprüche**

**1.** Verfahren zur Herstellung von Polyoxymethylendimethylether aus Methanol, umfassend die Kontaktreaktion von Methanol mit einem Oxidationsmittel in der Gegenwart eines Katalysators, **dadurch gekennzeichnet, dass** der Katalysator zumindest eine Metallkomponente der Gruppe VIB in einer Menge von 0,5 bis 50 Gew.-% (als Metalloxid) und zumindest eine Metallkomponente der Gruppe VIII in einer Menge von 0,2 bis 20 Gew.-% (als Metalloxid) und zumindest ein Molekularsieb mit einer sauren katalytischen Aktivität in einer Menge von 40 bis 95 Gew.-% enthält, bezogen auf das Gesamtgewicht des Katalysators, und worin die Metallkomponente der Gruppe VIII im Katalysator Eisen ist, worin das Oxidationsmittel Sauerstoff oder eine Gasmischung aus Sauerstoff mit anderen Gasen ist, die für Methanol inert sind.

**2.** Verfahren nach Anspruch 1, **dadurch gekennzeichnet, dass** das Molekularsieb mit einer sauren katalytischen Aktivität ausgewählt ist aus der Gruppe bestehend aus Molekularsieb mit mesoporöser Struktur, makroporöser Struktur und einer Kombination davon, worin die Menge des Molekularsiebes 70 bis 90 Gew.-% ist, bezogen auf das Gesamtgewicht des Katalysators.

**3.** Verfahren nach Anspruch 2, **dadurch gekennzeichnet, dass** das Molekularsieb mit mesoporöser Struktur ZSM-5 Molekularsieb ist und das Molekularsieb mit der makroporösen Struktur Molekularsieb vom Y-Typ ist, insbesondere worin das Molekularsieb vom Y-Typ ausgewählt ist aus der Gruppe bestehend aus HY, REY, REHY, USY, REUSY und einer Kombination davon, mehr bevorzugt, worin das Molekularsieb vom Y-Typ ausgewählt ist aus der Gruppe bestehend aus REY, REHY, REUSY und einer Kombination davon.

**4.** Verfahren nach Anspruch 1, **dadurch gekennzeichnet, dass** der Katalysator weiterhin eine wärmeresistente anorganische Oxidmatrix in einer Menge von nicht mehr als 80 Gew.-%, bevorzugt nicht mehr als 60 Gew.-%, bezogen auf das Gesamtgewicht des Katalysators enthält, wobei bevorzugt die wärmeresistente anorganische Oxidmatrix ausgewählt ist aus der Gruppe bestehend aus Aluminiumoxid, Siliziumoxid, Siliziumoxid-Aluminiumoxid und einer Kombination davon.

**5.** Verfahren nach Anspruch 1, **dadurch gekennzeichnet, dass** die Bedingungen für die Kontaktreaktion eine Temperatur innerhalb des Bereiches von 50 bis 500°C, bevorzugt 100 bis 400°C, mehr bevorzugt 100 bis 300°C, einen Druck innerhalb eines Bereiches von 0,1 bis 10 MPa, bevorzugt 0,1 bis 5 MPa, mehr bevorzugt 0,1 bis 2 MPa und eine Massenraumgeschwindigkeit von zugeführtem Methanol innerhalb des Bereiches von 0,5 bis 50 $h^{-1}$, bevorzugt 3 bis 30 $h^{-1}$, mehr bevorzugt 8 bis 20 $h^{-1}$ sind und der Gehalt des sauren Oxidationsmittels so ausgewählt ist, dass das molare Verhältnis von Sauerstoff zu Methanol in der Kontaktreaktion 0,01 bis 0,5, bevorzugt 0,05 bis 0,3 ist.

**6.** Verfahren nach Anspruch 1, **dadurch gekennzeichnet, dass** das Gas, das für Methanol inert ist, ein oder mehrere Gase ist, ausgewählt aus Stickstoff und Inertgasen.

**7.** Verfahren nach Anspruch 6, **dadurch gekennzeichnet, dass** der Sauerstoffgehalt in der Gasmischung 1 bis 30% ist.

**8.** Verfahren nach Anspruch 1, **dadurch gekennzeichnet, dass** es ein Gasphasenverfahren zur Herstellung von Polyoxymethylendimethylether (DMMx) aus Methanol umfasst, umfassend das Mischen von Methanol und dem Oxidationsmittel zur Bildung eines binären gemischten Gases aus Methanol und dem Oxidationsmittel, Zuführen des gemischten Gases zu einem Reaktor, Durchleiten des gemischten Gases durch ein Katalysatorbett, zur Bildung einer Produktmischung, umfassend DMMx, und dass es einen Schritt zur Einstellung der Temperatur des Reaktors unter gesteuerter thermischer Bedingung umfasst.

**9.** Verfahren nach Anspruch 8, **dadurch gekennzeichnet, dass** der Schritt der Einstellung der Temperatur des Reaktors unter gesteuerter thermischer Bedingung das Steuern der Zyklusmenge von Nicht-DMMx-Bereichen in der Produktmischung enthält, zum Einstellen der Reaktionstemperatur.

**10.** Verfahren nach Anspruch 9, **dadurch gekennzeichnet, dass** die Steuerung der Zyklusmenge von Nicht-DMMx-Teilen in der Produktmischung das Einführen von Nicht-DMMx-Bereichen in der Produktmischung in das Katalysatorbett direkt von der oberen Seite des Reaktors oder an vielen Punkten in separaten Stufen und die Steuerung der Temperatur des Zyklusproduktes unter 0 bis 150°C umfasst.

**11.** Verfahren nach Anspruch 9, **dadurch gekennzeichnet, dass** das Verhältnis der Menge des Zyklusproduktes zum Ausgangsmethanol von 0,1 bis 100 ist.

**12.** Verfahren nach Anspruch 8, **dadurch gekennzeichnet, dass** der Schritt des Einstellens der Temperatur des Reaktors unter gesteuerter thermischer Bedingung das Kühlen mit Kühlmedium außerhalb des Reaktionsbettes umfasst, wobei das Kühlmedium bevorzugt Luft, Wasser oder Wärmetransferöl ist.

**13.** Verfahren nach Anspruch 8, **dadurch gekennzeichnet, dass** es weiterhin das Führen der Produktmischung, die DMMx umfasst, in einen Trennvorgang umfasst.

**14.** Verfahren nach Anspruch 8, **dadurch gekennzeichnet, dass** das Katalysatorbett ein Festbett ist.

**Revendications**

**1.** Procédé de préparation d'éther diméthylique de polyoxyméthylène à partir de méthanol, comprenant la réaction par contact de méthanol avec un oxydant en présence d'un catalyseur, **caractérisé en ce que** le catalyseur comprend au moins un composant métal du Groupe VIB dans une quantité de 0,5 à 50% en poids (en termes d'oxyde de métal) et au moins un composant métal du Groupe VIII dans une quantité de 0,2 à 20% en poids (en termes d'oxyde de métal), et au moins un tamis moléculaire ayant une activité catalytique acide dans une quantité de 40 à 95% en poids, sur la base du poids total du catalyseur, et le composant métal du Groupe VIII dans le catalyseur est le fer, dans lequel l'oxydant est l'oxygène ou un mélange gazeux d'oxygène avec d'autres gaz qui sont inertes pour le méthanol.

**2.** Procédé selon la revendication 1, **caractérisé en ce que** le tamis moléculaire ayant une activité catalytique acide est choisi parmi le groupe consistant en un tamis moléculaire ayant une structure mésoporeuse, une structure macroporeuse, et une combinaison de celles-ci, dans lequel la quantité du tamis moléculaire est 70 à 90% en poids sur la base du poids total du catalyseur.

**3.** Procédé selon la revendication 2, **caractérisé en ce que** le tamis moléculaire avec une structure mésoporeuse est le tamis moléculaire ZSM-5, et le tamis moléculaire avec une structure macroporeuse est un tamis moléculaire de type Y, préférablement le tamis moléculaire de type Y est choisi parmi le groupe consistant en HY, REY, REHY, USY, REUSY, et une combinaison de ceux-ci, plus préférablement le tamis moléculaire de type Y est choisi parmi le groupe consistant en REY, REHY, REUSY, et une combinaison de ceux-ci.

**4.** Procédé selon la revendication 1, **caractérisé en ce que** le catalyseur comprenant en outre une matrice d'oxyde inorganique résistante à la chaleur dans une quantité de pas plus de 80% en poids, préférablement pas plus de 60% en poids, sur la base du poids total du catalyseur, préférablement la matrice d'oxyde inorganique résistante à la chaleur est choisie parmi le groupe consistant en une alumine, une silice, une silice-alumine, et une combinaison de celles-ci.

**5.** Procédé selon la revendication 1, **caractérisé en ce que** les conditions pour la réaction par contact comprennent une température à l'intérieur de la plage de 50°C à 500°C, préférablement de 100 à 400°C, plus préférablement de 100 à 300°C, une pression à l'intérieur de la plage de 0,1 MPa à 10 MPa, préférablement de 0, 1 MPa à 5 MPa, plus préférablement de 0,1 MPa à 2 MPa, et une vitesse massique dans l'espace d'alimentation de méthanol à l'intérieur de la plage de 0,5 $h^{-1}$ à 50 $h^{-1}$, préférablement de 3 à 30 $h^{-1}$, plus préférablement de 8 à 20 $h^{-1}$, et la teneur de l'oxydant est choisie de telle manière que le rapport molaire de l'oxygène sur le méthanol dans la réaction par contact est 0,01 à 0,5, préférablement de 0,05 à 0, 3.

**6.** Procédé selon la revendication 1, **caractérisé en ce que** le gaz qui est inerte pour le méthanol est un ou plusieurs gaz choisi(s) parmi l'azote et des gaz inertes.

**7.** Procédé selon la revendication 6, **caractérisé en ce que** la teneur en oxygène dans le mélange gazeux est 1 à 30%.

**8.** Procédé selon la revendication 1, **caractérisé en ce qu'**il comprend un processus en phase gazeuse de préparation d'éther diméthylique de polyoxyméthylène (DMMx) à partir de méthanol, comprenant le mélange de méthanol et de l'oxydant pour former un gaz mixte binaire de méthanol et de l'oxydant, l'alimentation du gaz mixte dans un réacteur, le passage du gaz mixte à travers un lit de catalyseur pour former un mélange produit contenant du DMMx et qu'il comprend une étape d'ajustement de la température du réacteur sous une condition thermique contrôlée.

**9.** Procédé selon la revendication 8, **caractérisé en ce que** l'étape d'ajustement de la température du réacteur sous une condition thermique contrôlée comprend le contrôle de la quantité dans le cycle des parties non DMMx dans le mélange produit pour ajuster la température de réaction.

**10.** Procédé selon la revendication 9, **caractérisé en ce que** le contrôle de la quantité dans le cycle des parties non DMMx dans le mélange produit inclut l'incorporation de parties non DMMx dans le mélange produit dans un lit de catalyseur directement à partir du haut du réacteur ou en des points multiples dans un étage séparé, et le contrôle de la température du produit cyclé en-dessous de 0 à 150°C.

**11.** Procédé selon la revendication 9, **caractérisé en ce que** le rapport de la quantité du produit cyclé sur le méthanol de départ est de 0,1 à 100.

**12.** Procédé selon la revendication 8, **caractérisé en ce que** l'étape d'ajustement de la température du réacteur sous une condition thermique contrôlée comprend un refroidissement avec un agent de refroidissement à l'extérieur du lit de réaction, préférablement l'agent de refroidissement est l'air, l'eau ou une huile de transfert de chaleur.

**13.** Procédé selon la revendication 8, **caractérisé en ce qu'**il comprend en outre l'alimentation du mélange produit contenant du DMMx dans une procédure de séparation.

**14.** Procédé selon la revendication 8, **caractérisé en ce que** le lit de catalyseur est un lit fixe.

Figure1

EP 2 228 359 B1

Figure 2

EP 2 228 359 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6166266 A
- EP 1505049 A1
- WO 0029364 A
- US 20050154226 A
- DE 102005027701
- CN 1187462 A
- CN 1121979 C
- CN 1257840 C
- CN 1005387 B
- CN 1069553 C
- CN 1205915 A
- CN 10610976 A
- GB 3382004 A